# FASCICULE DE BREVET EUROPEEN

(11) **EP 2 090 303 B1**
(45) Date de publication et mention de la délivrance du brevet: **01.05.2013**
(21) Numéro de dépôt: 09290099.2
(22) Date de dépôt: 12.02.2009
(51) Int. Cl.: A61K 31/165, A61K 31/55, A61P 9/10, A61P 9/04

(54) **Association d'un inhibiteur du courant if sinusal et d'un bêta-bloquant**
Verbindung eines LF-Sinusstromhemmers und eines Betablockers
Association of a sinus current inhibitor if and a beta blocker

(30) Priorité: 14.02.2008 FR 0800800
(43) Date de publication de la demande: 19.08.2009
(60) Demande divisionnaire: 11008255.9
(73) Titulaire: Les Laboratoires Servier, 92284 Suresnes Cedex (FR)
(72) Inventeur: Lerebours, Guy, 92300 Levallois-Perret (FR); Calvet, Jean-Henri, 75003 Paris (FR)
(74) Mandataire: Bestel, Delphine

(56) Documents cités:
- EP-A- 1 800 678
- EP-A- 1 800 683
- LECHAT P: "De l'ischémie à l'insuffisance cardiaque: la fréquence cardiaque - acteur ou marqueur?" THÉRAPIE, vol. 59, no. 5, septembre 2004 (2004-09), pages 485-489, XP009070208
- THE BEAUTIFUL STUDY GROUP: "The BEAUTIFUL study: Randomized trial of ivabradine in patients with stable coronary artery disease and left ventricular systolic dysfunction - Baseline characteristics of the study population" CARDIOLOGY, vol. 110, no. 4, 12 décembre 2007 (2007-12-12), pages 271-282, XP009104278
- MANGIN L, SWYNGHEDAUW B, BENIS A, THIBAULT N, LEREBOURS G, CARRÉ F: "Relationships between heart rate and heart rate variability: Study in conscious rats" JOURNAL OF CARDIOVASCULAR PHARMACOLOGY, vol. 32, 1998, pages 601-607, XP009104277
- KLEIN WERNER W ET AL: "Efficacy of monotherapy compared with combined antianginal drugs in the treatment of chronic stable angina pectoris: a meta-analysis.", CORONARY ARTERY DISEASE DEC 2002 LNKD- PUBMED:12544718, vol. 13, no. 8, December 2002 (2002-12), pages 427-436, ISSN: 0954-6928

## Description

La présente invention concerne l'association de l'ivabradine en tant qu'inhibiteur du courant If sinusal et de l'atérolol ou du bisopilol en tant que bêta-bloquant ou β-bloquant, ainsi que les compositions pharmaceutiques qui les contiennent.

Plus particulièrement, la présente invention concerne une association fixe dun inhibiteur du courant If sinusal qui est livabradine ou 3-{3-[{[(7S)-3,4-diméthoxybicyclo[4.2.0]octa-1,3,5-trién-7-yl]méthyl}(méthyl)amino]propyl}-7,8-di-méthoxy-1,3,4,5-tétrahydro-2H-3-benzazépin-2-one de formule (I) : 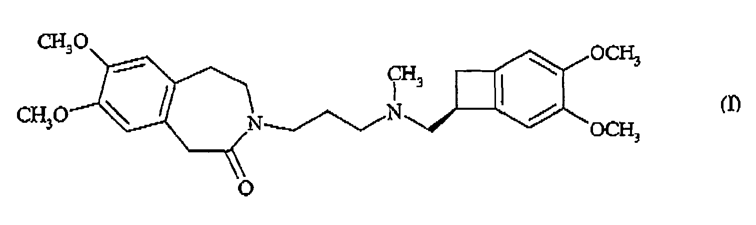 ainsi que ses hydrates, formes cristallines et sels d'addition à un acide pharmaceutiquement acceptable, et de l'atérolol ou du bisopilol en tant que β-bloquant.

De manière préférée, la présente invention a pour objet une association entre l'aténolol ou un de ses hydrates, formes cristallines ou sels d'addition à un acide pharmaceutiquement acceptable, et l'ivabradine chlorydrate ou un de ses hydrates ou formes cristallines en tant quinhibiteur du courant If sinusal.

Les inhibiteurs du courant If sinusal et plus particulièrement l'ivabradine, ainsi que ses hydrates, formes cristallines et ses sels d'addition à un acide pharmaceutiquement acceptable, et plus particulièrement son chlorhydrate, possèdent des propriétés pharmacologiques et thérapeutiques très intéressantes, notamment des propriétés chronotropes négatives (réduction de la fréquence cardiaque), qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0534 859.

Les β-bloquants ou β-bloqueurs sont des molécules inhibant les récepteurs adrénergiques β. Les β-bloquants ont pour effet de ralentir le c□ ur en diminuant la pente de dépolarisation, de diminuer le travail du c□ ur et de réduire ses besoins en oxygène. En conséquence, les β-bloquants sont utilisés dans le traitement de langor, en particulier en prévention des crises. De manière paradoxale en apparence, les β-bloquants sont utilisés dans le traitement de linsuffisance cardiaque.

De manière préférée, les β-bloquants selon linvention sont des β -bloquants cardio-sélectifs cest à dire bloquant préférentiellement les récepteurs β 1-cardiaques. Le caractère cardio-sélectif permet de diminuer les effets secondaires en particulier la vasoconstriction périphérique et la bronchoconstriction,
Les β-bloquants cardio-sélectifs sont l'aténolol, ou le bisoprolol.

La littérature et les leaders dopinion dans le domaine des maladies cardiovasculaires concluent dune manière générale à la difficulté de la démonstration de la supériorité des associations β-bloquant plus une autre classe de composés anti-angor versus un β-bloquant en monothérapie dans le traitement de langor stable.

La présente invention met en évidence de manière surprenante l'ivabradine est capable de potentialiser les effetsde l'atérolol et du bisoprolol sur laugmentation de la capacité à lexercice. Cette augmentation de la capacité à l'exercice est donc liée à une synergie entre les principes actifs, i.e. de l'ivabradine en tan qu'inhibiteur du courant If sinusal et de l'atérolol et du bisoprolol en tant que de β-bloquant.

Dans les compositions pharmaceutiques selon l'invention, les quantités dinhibiteur du courant If sinusal et de β-bloquant sont adaptées à la nature de ces principes actifs et leurs proportions relatives sont donc variables en fonction desdits principes actifs.

Les compositions pharmaceutiques selon linvention présentent des proportions comprises entre 1,6% et 80% de la masse totale des principes actifs pour linhibiteur du courant If sinusal et entre 20% et 98% de la masse totale des principes actifs pour le β-bloquant.

Lorsque linhibiteur du courant If sinusal est livabradine sous forme de sel de chlorydrate et que le β-bloquant est latenolol les pourcentages préférés pour cette association sont autour de 25% divabradine sous forme de sel de chlorydrate contre 75% datenolol.

La présente invention concerne également les compositions pharmaceutiques comprenant comme principe actif une association de l'ivabradine en tan qu'inhibiteur du courant If sinusal et de l'atérolol et du bisoprolol en tant que de β-bloquant, éventuellement sous forme de sels pharmaceutiquement acceptables, seuls ou avec un ou plusieurs véhicules ou excipients inertes, non toxiques et appropriés.

Dans les compositions pharmaceutiques selon linvention, la fraction massique en principes actifs (masse des principes actifs sur la masse totale de la composition) est avantageusement comprise entre 5 et 50%.

En ce qui concerne les excipients pharmaceutiquement acceptables, on peut citer à titre non limitatif les liants, les diluants, les délitants, les stabilisants, les conservateurs, les lubrifiants, les odorants, les aromatisants ou les édulcorants.

A titre d'exemple et de manière non limitative, on peut citer :
- pour les diluants : le lactose, le dextrose, le sucrose, le mannitol, le sorbitol, la cellulose, la glycérine ;
- pour les lubrifiants : la silice, le talc, l'acide stéarique et ses sels de magnésium et de calcium, le polyéthylène glycol ;
- pour les liants : le silicate d'aluminium et de magnésium, l'amidon, la gélatine, la tragacanthe, la méthylcellulose, la carboxyméthylcellulose de sodium et la polyvinylpyrrolidone.

Parmi les compositions pharmaceutiques selon l'invention, on retiendra plus particulièrement celles qui conviennent pour l'administration par voie orale, parentérale et notamment intraveineuse, per ou transcutanée, nasale, rectale, perlinguale, oculaire, respiratoire et plus spécifiquement les comprimés simples ou dragéifiés, les comprimés sublinguaux, les gélules, les glossettes, les capsules, les tablettes, les préparations injectables, les aérosols, les gouttes oculaires ou nasales, les suppositoires, les crèmes, pommades ou gels dermiques.

La voie dadministration préférée est la voie orale et les compositions pharmaceutiques correspondantes peuvent permettre la libération instantanée ou différée des principes actifs.

Les compositions pharmaceutiques préférées sont les comprimés.

La posologie est adaptable selon la nature et la sévérité de l'affection, la voie d'administration ainsi que l'âge et le poids du patient. Dans les compositions selon l'invention, elle séchelonne entre 25 et 150 mg pour le β -bloquant et entre 2.5 et 30 mg dinhibiteur du courant If sinusal en une ou plusieurs prises par 24 heures. De préférence, lorsque linhibiteur du courant If sinusal est livabradine la dose dadministration est comprise entre 5 et 7.5 mg deux fois par jour (b.i.d). Lorsque le β-bloquant est latenolol la dose dadministration journalière est, de préférence, de 50 mg en une prise.

Enfin, les compositions pharmaceutiques selon linvention sont utiles pour la fabrication de médicament destiné au traitement de langine de poitrine, de lischémie et de linsuffisance cardiaque.

Les exemples de composition ci-dessous sont donnés à titre non limitatif.

### Comprimés de ivabradine / atenolol :

**Exemple 1 :**

| Constituants | Quantité (mg) |
|---|---|
| Ivabradine, chlorydrate | 5 |
| atenolol | 50 |
| silice colloïdale hydrophobe | 0.4 |
| amidon | 6 |
| stéarate de magnésium | 2 |
| cellulose microcristalline | 50 |
| lactose | 86.6 |
| Pour un comprimé terminé à | 200 |

**Exemple 2 :**

| Constituants | Quantité (mg) |
|---|---|
| Ivabradine, chlorydrate | 7.5 |
| atenolol | 50 |
| silice colloïdale hydrophobe | 0.4 |
| amidon | 6 |
| stéarate de magnésium | 2 |
| cellulose microcristalline | 50 |
| lactose | 84.1 |
| Pour un comprimé terminé à | 200 |

### Etude clinique :

Les patients sélectionnés dans cette étude clinique sont des hommes et des femmes de 18 à 75 ans atteints dangine de poitrine depuis au moins 3 mois et traités par des β-bloquants tel que latenolol (50mg o.d.) ou tout autre β-bloquant à un dosage équivalent depuis au moins 3 mois. Ces patients présentent en dépit de leur traitement un test positif de tolérance à lexercice (ETT) et des symptômes quotidiens dangine de poitrine.

Cette étude clinique internationale, en double aveugle et en groupe parallèle, a été réalisée sur 889 patients. Les patients recevant tous l'atenolol sont randomisés en deux groupes :
- le premier groupe reçoit, en plus du β-bloquant, de livabradine (chlorhydrate) à la dose de 5mg deux fois par jour pendant deux mois puis à la dose de 7.5mg deux fois par jour pendant encore deux mois ;
- le deuxième groupe reçoit, en plus du β-bloquant, un placebo durant 4 mois.

Les patients sont soumis à un test de tolérance à lexercice sur tapis roulant (ETT) selon le protocole de Bruce avant la prise divabradine (sous β -bloquant seul) et après 4 mois de traitement par lassociation entre de livabradine et l'atenolol. Les principaux paramètres mesurés au cours de lépreuve deffort sont :
- la durée totale de lexercice (TED), le TED inclut le temps darrêt du tapis soit une durée denviron 10 secondes à compter de la demande darrêt de lexercice par le patient;
- le temps pour lequel la douleur « angine de poitrine » oblige le patient à arrêter lexercice (TLA) ;
- temps dapparition de la douleur « angine de poitrine » (TAO) ;
- temps dapparition sur lenregistrement électrocardiographique dun sous décalage segment ST de 1 mm (TST), reflétant lischémie, et correspondant au signe électrique de souffrance du muscle cardiaque.

Ces mesures sont effectuées au creux de lactivité de lassociation de principes actifs soit 12±1 heures et 24±2 heures après la prise des principes actifs.
Lassociation ivabradine / β-bloquant selon l'invention permet une bonne acceptabilité ainsi qu'une bonne sécurité d'emploi selon les données du recueil de la compliance.

Les patients ont en moyenne 60±8 ans. A lentrée dans létude, la pression artérielle moyenne systolique / diastolique et la fréquence cardiaque des patients au repos sont en moyenne respectivement : 127±12 / 78±7 mmHg et 67±7 battements par minute. Après 4 mois de traitement par l'association, la fréquence cardiaque est diminuée de 9±10bpm β-bloquant + placebo 1±10bpm).
Les améliorations moyennes des différents paramètres mesurés durant le test dexercice sur tapis roulant entre la valeur mesurée avant le début du traitement par ivabradine et la fin de la période de traitement sont consignés dans le tableau ci-dessous :

| | β-bloquant + ivabradine (n=441) | β-bloquant + placebo (n=434) | Valeur de p |
|---|---|---|---|
| TED | 24±65 | 8±64 | p< 0.001 |
| TAO | 49±83 | 23±79 | p< 0.001 |
| TLA | 26±66 | 9±64 | p< 0.001 |
| TST | 46±93 | 15±87 | p< 0.001 |

### Amélioration des paramètres (en secondes, moyenne ± SD)

Cette étude montre que livabradine est capable daméliorer la tolérance à lexercice de patients recevant déjà une dose standard de β-bloquants. Le niveau élevé de la compliance (99% chez les patients recevant de livabradine) et le faible pourcentage dévénements indésirables sérieux montrent lacceptabilité ainsi que la sécurité de lassociation ivabradine plus β-bloquant en comparaison au β-bloquant en monothérapie.

## Revendications

1. Association d'un inhibiteur du courant I_{f} sinusal et d'un β-bloquant **caractérisée en ce que** l'inhibiteur du courant If sinusal est l'ivabradine et le β-bloquant est l'aténolol ou le bisoprolol.

2. Association selon la revendication 1, **caractérisée en ce que** l'inhibiteur du courant I_{f} sinusal est l'ivabradine ou 3-{3-[{[(7S) -3,4-diméthoxybicyclo[4.2.0]octa-1,3,5-trién-7-yl]méthyl}(méthyl)amino]propyl}-7,8-di-méthoxy-1,3,4,5-tétrahydro-2*H*-3-benzazépin-2-one, ou un de ses hydrates, formes cristallines ou sels d'addition à un acide pharmaceutiquement acceptable.

3. Association selon l'une des revendications 1 ou 2, **caractérisée en ce que** l'inhibiteur du courant I_{f} sinusal est l'ivabradine ou 3-{3-[{[(7S) -3,4-diméthoxybicyclo[4.2.0]octa-1,3,5-trién-7-yl]méthyl}(méthyl)amino]propyl}-7,8-di-méthoxy-1,3,4,5-tétrahydro-2*H*-3-benzazépin-2-one, chlorhydrate ou un de ses hydrates ou formes cristallines.

4. Association selon la revendication 1, **caractérisée en ce que** le β-bloquant est l'aténolol, ou un de ses hydrates, formes cristallines ou sels d'addition à un acide pharmaceutiquement acceptable.

5. Association selon la revendication 1, **caractérisée en ce qu'**elle contient l'ivabradine ou un de ses hydrates, formes cristallines ou sels d'addition à un acide pharmaceutiquement acceptable, et l'aténolol ou un de ses hydrates, formes cristallines ou sels d'addition à un acide pharmaceutiquement acceptable.

6. Composition pharmaceutique comprenant comme principe actif une association selon l'une des revendications 1 à 5 seul ou en combinaison avec un ou plusieurs excipients pharmaceutiquement acceptables.

7. Composition pharmaceutique selon la revendication 6 utile pour la fabrication d'un médicament pour le traitement de l'angine de poitrine, de l'ischémie et de l'insuffisance cardiaque.

8. Utilisation d'une association selon l'une des revendications 1 à 5 pour la fabrication d'un médicament pour le traitement de l'angine de poitrine, de l'ischémie et de l'insuffisance cardiaque.

9. Utilisation d'une composition pharmaceutique selon l'une des revendications 6 à 7 pour la fabrication d'un médicament pour le traitement de l'angine de poitrine, de l'ischémie et de l'insuffisance cardiaque.

## Claims

1. Association of a sinus node I_{f} current inhibitor and a β-blocker, **characterised in that** the sinus node I_{f} current inhibitor is ivabradine and the β-blocker is atenolol or bisoprolol.

2. Association according to claim 1, **characterised in that** the sinus node I_{f} current inhibitor is ivabradine, or 3-{3-[{[(7S)-3,4-dimethoxybicyclo[4.2.0]-octa-1,3,5-trien-7-yl]methyl}(methyl)amino]propyl}-7,8-dimethoxy-1,3,4,5-tetrahydro-2*H*-3-benzazepin-2-one, or one of its hydrates, crystalline forms or addition salts with a pharmaceutically acceptable acid.

3. Association according to either claim 1 or claim 2, **characterised in that** the sinus node I_{f} current inhibitor is ivabradine - or 3-{3-[{[(7S)-3,4-dimethoxybicyclo[4.2.0]octa-1,3,5-trien-7-yl]methyl}(methyl)amino]propyl}-7,8-dimethoxy-1,3,4,5-tetrahydro-2*H*-3-benzazepin-2-one - hydrochloride or one of its hydrates or crystalline forms.

4. Association according to claim 1, **characterised in that** the β-blocker is atenolol or one of its hydrates, crystalline forms or addition salts with a pharmaceutically acceptable acid.

5. Association according to claim 1, **characterised in that** it comprises ivabradine, or one or its hydrates, crystalline forms or addition salts with a pharmaceutically acceptable acid, and atenolol, or one of its hydrates, crystalline forms or addition salts with a pharmaceutically acceptable acid.

6. Pharmaceutical composition comprising, as active ingredient, an association according to one of claims 1 to 5, on its own or in combination with one or more pharmaceutically acceptable excipients.

7. Pharmaceutical composition according to claim 6 for use in the manufacture of a medicament for the treatment of angina pectoris, ischaemia and heart failure.

8. Use of an association according to one of claims 1 to 5 in the manufacture of a medicament for the treatment of angina pectoris, ischaemia and heart failure.

9. Use of a pharmaceutical composition according to one of claims 6 to 7 in the manufacture of a medicament for the treatment of angina pectoris, ischaemia and heart failure.

## Patentansprüche

1. Kombination aus einem I_{f}-Sinusstrom-Hemmer und einem β-Blocker, **dadurch gekennzeichnet, dass** der I_{f}-Sinusstrom-Hemmer Ivabradin und der β-Blocker Atenolol oder Bisoprolol ist.

2. Kombination nach Anspruch 1, **dadurch gekennzeichnet, dass** der I_{f}-Sinusstrom-Hemmer Ivabradin oder 3-{3-[{[(7S)-3,4-Dimethoxybicyclo[4.2.0]octa-1,3,5-trien-7-yl]-methyl}(methyl)-amino]-propyl}-7,8-dimethoxy-1,3,4,5-tetrahydro-2*H*-3-benzazepin-2-on ist oder eines seiner Hydrate, Kristallformen oder Additionssalze mit einer pharmazeutisch annehmbaren Säure.

3. Kombination nach einem der Ansprüche 1 oder 2, **dadurch gekennzeichnet, dass** der I_{f}-Sinusstrom-Hemmer Ivabradin oder 3-{3-[{[(7S)-3,4-Dimethoxybicyclo[4.2.0]-octa-1,3,5-trien-7-yl]-methyl} (methyl)-amino]-propyl}-7,8-dimethoxy-1,3,4,5-tetrahydro-2*H*-3-benzazepin-2-on, sein Hydrochlorid oder eines seiner Hydrate oder Kristallformen ist.

4. Kombination nach Anspruch 1, **dadurch gekennzeichnet, dass** der β-Blocker Atenolol ist oder eines seiner Hydrate, Kristallformen oder Additionssalze mit einer pharmazeutisch annehmbaren Säure.

5. Kombination nach Anspruch 1, **dadurch gekennzeichnet, dass** sie Ivabradin oder eines seiner Hydrate, Kristallformen oder Additionssalze mit einer pharmazeutisch annehmbaren Säure und Atenolol oder eines seiner Hydrate, Kristallformen oder Additionssalze mit einer pharmazeutisch annehmbaren Säure enthält.

6. Pharmazeutische Zubereitung enthaltend als Wirkstoff eine Kombination gemäß einem der Ansprüche 1 bis 5 allein oder in Kombination mit einem oder mehreren pharmazeutisch annehmbaren Hilfsstoffen.

7. Pharmazeutische Zubereitung nach Anspruch 6, nützlich für die Herstellung eines Arzneimittels zur Behandlung von Angina pectoris, Ischämie und Herzinsuffizienz.

8. Verwendung einer Kombination nach einem der Ansprüche 1 bis 5 für die Herstellung eines Arzneimittels zur Behandlung von Angina pectoris, Ischämie und Herzinsuffizienz.

9. Verwendung einer pharmazeutischen Zubereitung nach einem der Ansprüche 6 bis 7 zur Herstellung eines Arzneimittels für die Behandlung von Angina pectoris, Ischämie und Herzinsuffizienz.
